# EUROPEAN PATENT APPLICATION

(11) **EP 4 293 339 A1**
(43) Date of publication of application: **20.12.2023**
(21) Application number: 22179574.3
(22) Date of filing: 17.06.2022
(51) Int. Cl.: G01N 1/34, C07K 1/14, G01N 1/40, G01N 30/00

(54) **SAMPLE PREPARATION SYSTEM AND METHOD FOR PREPARING A SAMPLE USING THE SAMPLE PREPARATION SYSTEM**

(71) Applicant: The Automation Partnership (Cambridge) Ltd., Hertfordshire SG8 5WY (GB)
(72) Inventor: BARGH, Neil, Royston, Hertfordshire SG8 5WY (GB); BETTS, John, Royston, Hertfordshire SG8 5WY (GB)
(74) Representative: Müller-Boré & Partner Patentanwälte PartG mbB

(57) **Abstract**

The present invention relates to a sample preparation system and a method for preparing a sample using the sample preparation system.

## Description

The present invention relates to a sample preparation system and a method for preparing a sample using the sample preparation system.

In order to yield a target substance such as a protein by purification, the sample containing the target substance usually needs to be subjected to a clarification step prior to purification which removes contaminants including host cell-derived contaminants, such as whole cells, cell debris, host cell nucleic acids and host cell proteins, culture medium-derived contaminants, such as proteinaceous and non-proteinaceous medium components, and vector-derived contaminants, including vector nucleic acids and viral vectors.

Clarification and purification are usually carried out in two separate devices, wherein a first device clarifies the sample containing the target substance and a second distinct device uses the clarified output from the first device as input for purification which is commonly provided to the second device manually. Therefore, the combination of clarification and purification of a sample is usually a rather slow and inefficient process which may lead to degradation of the sample.

In view of the above, the technical problem underlying the present invention is to provide a sample preparation system with which two kinds of unit operations such as clarification and purification can be carried out fast in one device for improving the efficiency of the respective process.

The solution to the above technical problem is achieved by the embodiments characterized in the claims.

In particular, in an aspect, the present invention provides a sample preparation system comprising:
a serial arrangement of a first means configured to being connected to a first consumable and a second means configured to being connected to a second consumable;
a first valve arranged between the first means and the second means;
a second valve arranged downstream of the second means;
a first line providing fluidic communication between the first means and the first valve;
a second line providing fluidic communication between the first valve and the second valve via the second means;
a third line providing fluidic communication directly between the first valve and the second valve configured to bypass the second means; and
a pump configured to move fluid within the sample preparation system.

According to the present invention, the sample preparation system comprises a serial arrangement of the first means and the second means. In particular, the second means is located downstream of the first means in the sample preparation system, and the first means and the second means are integrally connected with each other in the sample preparation system. The term "integrally connected" used herein means arranged within one device, i.e. within the sample preparation system, and not arranged in separate devices. Since the sample preparation system of the present invention allows carrying out a first unit operation such as clarification and a second unit operation such as purification in combination in an automated way in one device, the respective method is fast and has a significantly improved process efficiency compared to methods which are performed in two separate devices.

The terms "automated" and "automatically" used herein means that the respective step can be carried out without intervention by a person. In contrast, the wording "manually" used herein means that the corresponding step is carried out by a person.

The first means of the sample preparation system is not particularly limited as long as it is configured to being connected to the first consumable. According to an embodiment, the first means is configured to being connected to the first consumable and to support the first consumable. For example, the first means can be a first filter holder or an end part of the first line providing fluidic communication between the first means, i.e. the end part of the first line itself in this case, and the first valve which supports the first consumable from its downstream side. In case that the first consumable is supported by the end part of the first line from its downstream side, the first consumable is preferably supported on its upstream side by an end part of a line upstream of the first means which is connected to the first means.

Preferably, the first means is a first filter holder. In an embodiment, the first filter holder comprises a first clamp configured to clamp the first consumable on the upper face (i.e. upstream) and a second clamp configured to clamp the first consumable on the lower face (i.e. downstream). Thereby, the first consumable can be securely supported against the internal pressure during operation. According to an embodiment, each of the first clamp and the second clamp comprises an O ring which is configured to connect the flow path upstream and downstream of the first means when the O ring is in a compressed state (i.e. when the clamps are closed). Preferably, one of the first clamp and the second clamp is a static clamp, i.e. it does not move, while the other is a movable clamp. Thereby, the first consumable can be clamped by pressing the movable clamp against the static clamp. Preferably, the first clamp is the static clamp, and the second clamp is the movable clamp.

According to a preferred embodiment, the sample preparation system further comprises an input stack configured to hold at least one first consumable. Preferably, the input stack is configured to hold from 1 to 150 first consumables, more preferably from 1 to 50 first consumables.

Further, the second means is not particularly limited as long as it is configured to being connected to the second consumable. According to an embodiment, the second means is configured to being connected to the second consumable and to support the second consumable. For example, the second means can be a second filter holder or can correspond to parts of the second line providing fluidic communication between the first valve and the second valve.

In case that the second means corresponds to parts of the second line, the second line is interrupted between the first valve and the second valve, and the second consumable is located at the interruption and supported by a first end part of the interrupted second line on the upstream side and by a second end part of the interrupted second line on the downstream side.

According to another embodiment, wherein the second means is a second filter holder, the second line is interrupted between the first valve and the second valve, and the second filter holder is located at the interruption and connected to a first end part of the interrupted second line on the upstream side and to a second end part of the interrupted second line on the downstream side. In an embodiment, the second filter holder comprises a first clamp configured to clamp the second consumable on the upper face (i.e. upstream) and a second clamp configured to clamp the second consumable on the lower face (i.e. downstream). Thereby, the second consumable can be securely supported against the internal pressure during operation. According to an embodiment, each of the first clamp and the second clamp comprises an O ring which is configured to connect the flow path upstream and downstream of the second means when the O ring is in a compressed state (i.e. when the clamps are closed). Preferably, one of the first clamp and the second clamp is a static clamp, i.e. it does not move, while the other is a movable clamp. Thereby, the second consumable can be clamped by pressing the movable clamp against the static clamp. Preferably, the first clamp is the static clamp, and the second clamp is the movable clamp.

In the present invention, a first valve is arranged between the first means and the second means. The first valve is configured to direct fluid to the second line or the third line of the sample preparation system. According to an embodiment, in case that fluid is directed to the second line, the flow of fluid to the third line is blocked. According to an alternative embodiment, in case that fluid is directed to the third line, the flow of fluid to the second line is blocked. The first valve is not particularly limited. In an embodiment, the first valve can for example be a 3-way diaphragm valve which has three ports, and can be in one of two states, directing flow to one of its two outlets, i.e. the second line and the third line. In another embodiment, the first valve is a 3-way ball valve.

Further, a second valve is arranged downstream of the second means. The second valve is configured to direct fluid to a line downstream of the second valve. The second valve is not particularly limited. In an embodiment, the second valve can for example be a 3-way diaphragm valve which has three ports with two inlets connected to the second line and the third line, and which can be in one of two states, directing flow from one of its two inlets, i.e. the second line and the third line, to the line downstream of the second valve. In another embodiment, the second valve is a 3-way ball valve. Preferably, the line is the fourth line which will be described further below.

The pump used in the sample preparation system of the present invention is not particularly limited. The pump is configured to move fluid within the sample preparation system. For example, the pump may be a peristaltic pump, a diaphragm pump or a piston pump. In an embodiment, the pump is arranged upstream of the first means. Further, the pump may be arranged downstream of an input sample valve which will be described below. Moreover, the pump may be arranged downstream of process fluid input valves which will be described further below. Thereby, the flow of fluid within the sample preparation system can be easily controlled.

According to an embodiment of the present invention, the sample preparation system further comprises a consumable handling device connected to the first means configured to provide a first consumable to the first means and to receive a first consumable from the first means. The consumable handling device operates in an automated manner. Preferably, the consumable handling device is connected to the input stack, wherein the consumable handling device is configured to receive a first consumable from the first means and to provide the first consumable to a waste bin and to receive a first consumable from the input stack and to provide the first consumable to the first means automatically or vice versa. By using the consumable handling device, a new (unused) first consumable can be easily provided to the first means while a used first consumable can be readily removed automatically.

The consumable handling device is not particularly limited. In an embodiment, the consumable handling device is an index wheel. Preferably, the index wheel has an opening in its in-plane direction which conforms to the shape of the first consumable at a first position of the index wheel. According to an embodiment, the index wheel can be rotated, whereby a first consumable is received from the input stack and provided to the first means. Moreover, the index wheel can be rotated to receive a first consumable from the first means and to provide it to the waste bin. In an embodiment, the consumable handling device is a filter handling device.

In an embodiment of the present invention, the sample preparation system further comprises an input line providing fluidic communication between an input cup arranged upstream of the first means and the first means. According to an embodiment, the input sample valve is located downstream of the input cup and upstream of the first means on the input line. Therefore, the input sample valve divides the input line into a part being upstream of the input sample valve and into a part being downstream of the input sample valve. In the embodiment, the input sample valve is configured to direct fluid to the input line downstream of the input sample valve or to a waste vessel. According to an embodiment, in case that fluid is directed to the input line downstream of the input sample valve, the flow of fluid to the waste vessel is blocked. According to an alternative embodiment, in case that fluid is directed to the waste vessel, the flow of fluid to the input line downstream of the input sample valve is blocked. The input sample valve is not particularly limited. In an embodiment, the input sample valve can for example be a 3-way diaphragm valve which has three ports, and can be in one of two states, directing flow to one of its two outlets, i.e. the input line downstream of the input sample valve and the waste vessel. In another embodiment, the input sample valve is a 3-way ball valve. The input cup is configured to hold a fluid, preferably the sample to be prepared. According to an embodiment, the input cup has a volume of from 10 mL to 150 mL, preferably from 20 mL to 80 mL.

In an embodiment, the sample preparation system further comprises a pipetting robot that is configured to transfer a fluid such as the sample to be prepared from at least one input vessel to the input cup. In an embodiment, one input vessel has a volume of from 2 mL to 20 mL. The input vessel is not particularly limited and can be selected from a culture vessel, a spin tube and a multi-well plate.

The pipetting robot that can be used in the present invention is not particularly limited. According to an embodiment, the pipetting robot comprises a pipette tip connected to a flow-through pH electrode and to fluid input valves. The fluid input valves are configured to provide for example water, sodium hydroxide and/or a pH neutralisation buffer to the pipette tip. Preferably, the pipetting robot further comprises a pipette liquid sensor arranged on a pipetting robot line providing fluidic communication between the pipette tip, the pH electrode and the fluid input valves. The pipette liquid sensor is configured to detect whether liquid is present or not at its location. Moreover, the pipetting robot may comprise a syringe connected to the flow-through pH electrode.

In an embodiment, the sample preparation system further comprises a pipette wash station configured to receive the pipette tip of the pipetting robot. In particular, the pipette wash station may have walls that surround the pipette tip of the pipetting robot while the pipetting robot is disposed therein. Further, the pipette wash station may have an outlet at its bottom through which liquid can emerge. Specifically, the liquid can be provided by at least one of the fluid input valves of the pipetting robot and can be used to wash the pipette tip while the pipetting robot is disposed in the pipette wash station. The outlet may be connected to a waste vessel to which the liquid may be pumped by the use of a tip wash waste pump.

Due to the possible use of the pipetting robot, the type of input vessel is not particularly limited. Therefore, the pipetting robot provides flexibility in the selection of the input vessel such that the choice thereof only depends on the fluid to be handled such as the sample to be prepared.

Preferably, the consumable handling device comprises a connecting means configured to connect the input line and the first line for providing fluidic communication between the input line and the first line. Thereby, a flow-through path can be created between the input line and the first line. In case that the consumable handling device is the index wheel, the connecting means corresponds to a hole in the in-plane direction of the index wheel that extends from the first surface of the index wheel to the second surface of the index wheel at a second position of the index wheel. The index wheel can be rotated such that a first opening of the hole on the first surface of the index wheel connects to the input line and a second opening of the hole on the second surface of the index wheel connects to the first line.

According to an embodiment of the present invention, the sample preparation system further comprises a first liquid sensor arranged upstream of the first means. The first liquid sensor is configured to detect whether liquid is present or not at the position where the first liquid sensor is arranged. Preferably, the first liquid sensor is arranged downstream of the input sample valve and upstream of the first means. According to this configuration, the first liquid sensor can for example detect whether the input cup has emptied.

In an embodiment, the sample preparation system according to the present invention further comprises a second liquid sensor arranged downstream of the first means and upstream of the second means. The second liquid sensor is configured to detect whether liquid is present or not at the position where the second liquid sensor is arranged. Preferably, the second liquid sensor is arranged upstream of the first valve. According to this configuration, based on the detection result of the second liquid sensor, the first valve and the second valve can be switched to direct fluid through the second line via the second means or through the third line for bypassing the second means.

The sample preparation system according to the present invention may further comprise a first pressure sensor arranged upstream of the first means. The first pressure sensor is configured to detect the pressure of the sample preparation system at the position where the first pressure sensor is arranged. Preferably, the first pressure sensor is arranged downstream of the pump and upstream of the first means. In an embodiment, the first pressure sensor is arranged downstream of the pump and the first liquid sensor and upstream of the first means. Thereby, the flow of fluid to the first means can be easily controlled.

In an embodiment, the sample preparation system of the present invention further comprises a second pressure sensor arranged downstream of the first means and upstream of the second means. The second pressure sensor is configured to detect the pressure of the sample preparation system at the position where the second pressure sensor is arranged. Preferably, the second pressure sensor is arranged upstream of the first valve. According to this configuration, based on the measurement result of the second pressure sensor, the first valve and the second valve can be switched to direct fluid through the second line via the second means or through the third line for bypassing the second means.

In an embodiment, the sample preparation system further comprises a fourth line providing fluidic communication between the second valve and an output valve arranged downstream of the second valve. The output valve is configured to direct fluid to an output line providing fluidic communication between the output valve and an output cup or to a waste vessel line providing fluidic communication between the output valve and a waste vessel. According to an embodiment, in case that fluid is directed to the output line, the flow of fluid to the waste vessel line is blocked. According to an alternative embodiment, in case that fluid is directed to the waste vessel line, the flow of fluid to the output line is blocked. The output valve is not particularly limited. In an embodiment, the output valve can for example be a 3-way diaphragm valve which has three ports, and can be in one of two states, directing flow to one of its two outlets, i.e. the output line and the waste vessel line. In another embodiment, the output valve is a 3-way ball valve.

Preferably, the sample preparation system further comprises the output line providing fluidic communication between the output valve and the output cup. The output cup is configured to hold fluid that has been output by the sample preparation system. In an embodiment, the output cup has a volume of from 10 mL to 150 mL, preferably from 20 mL to 80 mL. According to an embodiment, the sample preparation system comprises a pipetting robot which is configured to transfer the fluid from the output cup to at least one output vessel. This pipetting robot might be the same as the pipetting robot described above that is configured to transfer the fluid from at least one input vessel to the input cup. In an embodiment, one output vessel has a volume of from 2 mL to 80 mL. The output vessel is not particularly limited and can be selected, for example, from a 15 mL centrifuge tube, a 50 mL centrifuge tube and a multi-well plate.

Due to the possible use of the pipetting robot, the type of output vessel is not particularly limited. Therefore, the pipetting robot provides flexibility in the selection of the output vessel.

The sample preparation system according to the present invention may further comprise at least one process fluid input valve arranged upstream of the first means and configured to provide a process fluid to the sample preparation system. The process fluid is not particularly limited and may be selected from, for example, water, phosphate-buffered saline (PBS), elution buffer, a low pH strip buffer and sodium hydroxide. The elution buffer is not particularly limited, and the choice thereof depends on the substance to be eluted. The elution buffer may, for example, be selected from a citrate buffer, a glycine/HCI buffer and an acetate buffer. Each of the process fluids may for example be stored in a bottle and connected to the process fluid input valve by a line.

In an embodiment, the sample preparation system according to the present invention may further comprise a target substance detecting sensor arranged downstream of the second means configured to detect a target substance. According to this configuration, a target substance emerging from the first means and/or the second means can be easily detected. In an embodiment, the target substance detecting sensor is further arranged downstream of the second valve. Preferably, the target substance detecting sensor is further arranged upstream of the output valve. Thereby, depending on the detection result of the target substance detecting sensor, the fluid can be directed to the output cup or the waste vessel. Specifically, in case that a target substance can be detected by the target substance detecting sensor, the output valve can be switched to direct the target substance or fluid containing the target substance to the output cup. In case that the target substance is not detected by the target substance detecting sensor, the output valve can be switched to direct the fluid to the waste vessel.

The target substance is not specifically limited. For example, the target substance may be any biomolecule of interest, such as proteins, such as antibodies, hormones, vaccines, nucleic acids, exosomes, and viruses, and virus-like particles. In a preferred embodiment, the target substance is an antibody, more preferably a monoclonal antibody (mAb), or fragments or derivatives thereof, or nanobodies. Examples of monoclonal antibodies are adalimumab, cetuximab, rituximab, infliximab, omalizumab, and denosumab. The target substance can be obtained, for example, from mammalian cells, bacteria cells or insect cells, media and cell lines, such as "Chinese hamster ovary cells" (CHO cells), HeLa, or human umbilical vein endothelial cells (HUVEC).

In a preferred embodiment, the target substance detecting sensor is a UV sensor. Specifically, by using a UV sensor, the target substance such as a protein can be easily detected.

The first consumable that can be connected to the first means is not particularly limited. Preferably, the first consumable is a first filter. More preferably, the first filter is a clarification filter. According to an embodiment, the clarification filter is a depth filter and/or a membrane, preferably a depth filter. The clarification filter is configured to separate contaminants from a sample. Preferably, the clarification filter is a cell culture clarification filter. The cell culture clarification filter is configured to separate cells and other contaminants from a sample. In an embodiment, the first consumable is a single use consumable. The term "single use consumable" used herein means that the consumable cannot be cost effectively recovered after it has been used due to e.g. filter blockage and is thrown away.

The sample to be prepared is not particularly limited as long as it contains a plurality of components, wherein at least one component of the plurality of components of the sample is one of the above-described target substances. The further (impurity) components (contaminants) are not particularly limited and may depend on the preparation conditions of the target substance. Examples of further components are aggregates, host cell proteins, deoxyribonucleic acid, as well as fragments and charge variants thereof.

The source of the sample is not specifically limited. For example, the sample can be obtained by applying any biological, biochemical, chemical, or pharmaceutical method. Thereby, the sample can be obtained by previously conducting purification methods applying different purification units. For example, the target substance can be produced by appropriate cell lines, such as CHO cell lines e.g. by perfusion cultivation.

The second consumable that can be connected to the second means is not particularly limited. Preferably, the second consumable is a second filter or a chromatography column. More preferably, the second consumable is a purification filter or a purification chromatography column. According to an embodiment, the purification filter is a membrane. In an embodiment, the purification chromatography column contains functionalized beads. Preferably, the second consumable is configured to bind and elute the target substance. In an embodiment, the second consumable is a protein purification filter or a protein purification chromatography column, more preferably, a protein purification filter. Preferably, the protein purification filter or the protein purification chromatography column is configured to bind and elute a protein, preferably an antibody. In an embodiment, the second consumable is a reusable consumable. The term "reusable consumable" used herein means that the consumable can be used for several runs of sample preparation before it needs to be thrown away. For example, the reusable consumable needs to be replaced after performing from 50 to 150 operations. According to an embodiment, the purification filter or the purification chromatography column comprises a binding compound that allows binding the target substance within the purification filter or the purification chromatography column, respectively. For example, the binding compound is fixed on/in the filter matrix of the purification filter. The binding compound may for example be protein A which can bind the target substance at a pH of 7 or higher. The target substance can then be eluted by using an elution buffer having a pH lower than 7.

In an embodiment, the sample preparation system further comprises a user interface touchscreen. The user interface touchscreen allows easy control and operation of the sample preparation system.

The sample preparation system of the present invention may further comprise a pH sensor arranged downstream of the second means. The pH sensor is configured to measure the pH of liquid at the location of the pH sensor. Preferably, the pH sensor is further arranged downstream of the second valve and upstream of the output valve. According to an embodiment, the sample preparation system further comprises a pH adjustment input line providing fluidic communication between a pH adjusting agent reservoir and the second line upstream of the second means. Thereby, the pH at the second consumable which may be connected to the second means can be easily controlled. In addition, the sample preparation system may further comprise an elution fraction line providing fluid communication between an elution fraction reservoir and the second line downstream of the second means.

The material of the lines of the present invention is not particularly limited. Preferably, the material can be selected from a plastic such as polyvinyl chloride, polypropylene, polyethylene, polytetrafluoroethylene, and fluorinated ethylene propylene. Thereby, the lines can provide flexibility and chemical resistance.

The sample preparation system according to the present invention may comprise more than one serial arrangement of the first means and the second means. In particular, each of these serial arrangements may have the same configuration as the serial arrangement described above. In case that more than one serial arrangement is present in the sample preparation system, the serial arrangements are arranged parallel to each other, and each of the serial arrangements has its own input cup and output cup to allow independent operation.

The sample preparation system of the present invention enables performing two kinds of unit operations such as clarification and purification in one device such that the respective method is fast and has an improved process efficiency compared to methods using separate devices. In particular, a target substance comprised in more than 20 input vessels containing unfiltered sample can for example be clarified and purified in less than two hours. Accordingly, the sample preparation system according to the present invention can provide a fast method of clarifying and purifying a sample such that sample degradation can be avoided. Further, due to the automated operation of the sample preparation system, it is possible to reduce the need for manual labor of the respective process. Moreover, the sample preparation system can yield a well-defined (i.e. representative) output sample containing the target substance which may be directly subjected to further analysis via various assays.

According to another aspect, the present invention provides a method for preparing a sample using the sample preparation system, wherein the method comprises at least one of the following steps:
(i) clarifying the sample by passing it through a first consumable connected to the first means to provide a clarified sample, wherein the first consumable is a clarification filter (hereinafter referred to as "clarification process (i)") and
(ii) purifying the sample by passing it through a second consumable connected to the second means to provide a purified sample, wherein the second consumable is a purification filter or a purification chromatography column (hereinafter referred to as "purification process (ii)".

The present invention provides a method for preparing a sample using the sample preparation system, wherein the method comprises the clarification process (i), the purification process (ii) or both the clarification process (i) and the purification process (ii).

The following detailed description of the clarification process (i) relates to a method for preparing a sample using the sample preparation system, wherein the method comprises the clarification process (i) but not the purification process (ii) while the following detailed description of the purification process (ii) relates to a method for preparing a sample using the sample preparation system, wherein the method comprises the purification process (ii) but not the clarification process (i). Details on the method for preparing a sample using the sample preparation system, wherein the method comprises both the clarification process (i) and the purification process (ii) will be given further below.

Any definition of a feature made in the context of the sample preparation system also applies to the respective feature of the method for preparing a sample using the sample preparation system, unless explicitly stated otherwise.

According to a preferred embodiment of the method for preparing a sample of the present invention, the sample is a cell culture comprising a protein, the clarification filter is a cell culture clarification filter, and the purification filter is a protein purification filter or the purification chromatography column is a protein purification chromatography column. The respective method using the sample preparation system in accordance with the present invention can both clarify and purify the sample more efficiently and faster compared to a method, wherein clarification and purification are carried out in two separate devices. Further, the method of the present invention can yield a well-defined (i.e. representative) output sample containing the target substance such as a protein, preferably an antibody, which may be directly subjected to further analysis via various assays.

### Clarification process (i)

According to an embodiment, at the beginning of the clarification process (i), a clarification filter is provided to the first means, wherein the clarification filter is new, i.e. unused (hereinafter referred to as "step (c1)"). In said step (c1), preferably, the clarification filter is provided by the consumable handling device from the input stack.

In another embodiment, a clarification filter may already be connected to the first means at the beginning of the clarification process (i). In both embodiments, it might be necessary to exchange the clarification filter during the clarification process (i) for avoiding blockage of the clarification filter and product loss. According to an embodiment, the clarification filter is automatically ejected from the first means and automatically replaced by a new clarification filter. Preferably, based on the measured backpressure of the clarification filter measured by the first pressure sensor, or the pressure difference between the first pressure sensor and the second pressure sensor, and the pumping speed, a decision can be made to automatically eject the clarification filter from the first means and automatically replace it by a new clarification filter.

Then, the clarification filter is preferably clamped such that the upper face and the lower face of the clarification filter are supported against the internal pressure during filtration (i.e. "step (c2)").

Further, an air pressure test can be performed on the sample preparation system and the clarification filter before the sample to be clarified is pumped to the clarification filter (i.e. "step (c3)"). In particular, in said step (c3), the first valve and the second valve are set to close the flow path and the pump pumps air into the sample preparation system to reach a preset pressure. According to an embodiment, the preset pressure is from 0.1 bar to 4 bar, preferably from 0.5 bar to 1 bar. Then, the pressure is monitored for a few seconds. At the end of the air pressure test, the pressure is released by setting the first valve, the second valve and the output valve such that the pressure is released to the waste vessel via the third line.

After the optional air pressure test, the sample to be clarified can be provided to the input cup of the sample preparation system (i.e. "step (c4)a"). As an alternative, the sample to be clarified can also be provided to the input cup of the sample preparation system before any of steps (c1) to (c3) (i.e. "step (c4)b"). Preferably, the pipetting robot can provide the sample to be clarified to the input cup by transferring the sample to be clarified from at least one input vessel to the input cup of the sample preparation system.

Then, the sample to be clarified can be pumped from the input cup to the clarification filter (i.e. "step (c5)"). According to an embodiment of said step (c5), the sample to be clarified passes on its way from the input cup to the clarification filter at least the input sample valve, the first liquid sensor and the first pressure sensor.

Further, the sample is clarified by passing it through the clarification filter connected to the first means (hereinafter referred to as "step (c6)").

The clarified sample can then be directed to the output cup (hereinafter referred to as "step (c7)"). According to an embodiment of said step (c7), the clarified sample passes from the downstream side of the clarification filter to the output cup at least the second pressure sensor, the second liquid sensor, the third line, the target substance detecting sensor and the output valve.

In case that the input cup has emptied, the first liquid sensor can detect that the sample to be clarified has run out (i.e. "step (c8)"). Specifically, the first liquid sensor can detect whether the sample to be clarified previously provided to the input cup has already passed the location where the first liquid sensor is arranged.

After step (c8), a rinsing step can be automatically carried out (i.e. "step (c9)"). In particular, a rinsing liquid such as PBS can be provided to the sample preparation system by a process fluid input valve arranged upstream of the first means. Thereby, sample to be clarified still present upstream of the first means can be directed to the first means and sample within the clarification filter can be rinsed through the clarification filter. Preferably, the rinsing liquid which passed through the clarification filter containing clarified sample is directed to the output cup.

After step (c8) and/or step (c9), a step of pumping air is preferably carried out (i.e. "step (c10)"). Specifically, air can be provided to the sample preparation system by a process fluid input valve arranged upstream of the first means. Air pushes the sample to be clarified or rinse liquid still present on the upper flow field of the clarification filter and already partly clarified sample or rinse liquid still contained within the clarification filter through the clarification filter. The thereby yielded clarified sample can be directed to the output cup. By applying step (c10), product recovery can be increased. The air pressure used is for example in a range of from 0.5 bar to 2 bar. Thereby, the air pressure is below the bubble point of the clarification filter such that the creation of foam can be avoided. Typically, the bubble point of a clarification filter is greater than 3 bar. After a waiting time of for example from 0 s to 5 s, the air pressure is released by reversing the pump. The measurement of the bubble point is well known to the skilled person. For example, the bubble point can be measured according to ISO 2942 or ASTM F316-03.

Then, the clarification filter can be ejected from the first means (i.e. "step (c11)"). In case that the first means is the first filter holder, and the clarification filter is clamped by the first clamp and the second clamp, the clarification filter is unclamped before the clarification filter is ejected. The ejected clarification filter can be received by the consumable handling device and provided to the waste bin.

Further, according to an embodiment, the connecting means comprised by the consumable handling device connects the input line and the first line for providing fluidic communication between the input line and the first line such that the liquid path is closed (i.e. "step (c12)"). This can for example be achieved by using the index wheel as the consumable handling device and rotating the index wheel to the second position of the index wheel where a through fluidic passage is located which can then connect to the input line and the first line.

Afterwards, clarified sample and/or rinsing liquid containing the target substance that is present downstream of the first means can be directed to the output cup by pumping air (i.e. "step (c13)").

In an embodiment of the present invention, the output sample present in the output cup can be transferred to at least one output vessel (i.e. "step (c14)"). This step can, for example, be carried out by the pipetting robot.

Optionally, the sample preparation system can then be cleaned by pumping cleaning liquids such as a buffer, sodium hydroxide and/or water through the sample preparation system from process fluid input valves arranged upstream of the first means to the output cup, then in reverse from the output cup to the input cup (i.e. "step (c15)"). The resulting liquid can then be directed to a waste vessel by the use of a waste pump.

### Purification process (ii)

The purification filter or the purification chromatography column used in the purification process (ii) is preferably already present in the sample preparation system before the purification process (ii) begins. The purification filter or the purification chromatography column can for example be provided manually to the sample preparation system. In another embodiment, the purification filter or the purification chromatography column can be provided in an automated manner to the sample preparation system.

According to a preferred embodiment, the purification filter or the purification chromatography column is in an equilibrated state. The term "equilibrated state" used herein means that the purification filter or the purification chromatography column is in a state which allows binding the target substance. This state can for example be achieved by bringing the purification filter or the purification chromatography column to a certain pH such as 7 or higher while containing a binding compound. In case that the purification filter or the purification chromatography column is not in an equilibrated state, an equilibration buffer such as PBS can be pumped through the purification filter or the purification chromatography column. This step can be carried out at any point of the purification process (ii) before the below-described step (p3).

Further, according to an embodiment, excess air can be removed from the upstream side of the purification filter or the purification chromatography column for avoiding an air-lock blockage of the purification filter or the purification chromatography column. In particular, a buffer such as PBS can be pumped to the output cup, and then the pump is reversed to pull the buffer through the purification filter or the purification chromatography column from its outlet to the inlet while monitoring and controlling the negative pressure at the inlet of the purification filter or the purification chromatography column by the second pressure sensor. In an embodiment, the pressure is controlled to be 0.4 bar absolute or higher. Then, after the equilibration step and/or the step of removing excess air from the purification filter or the purification chromatography column, the input cup, the output cup and the lines of the sample preparation system except the second line can be drained.

According to an embodiment, at the beginning of the purification process (i), a flow-through path within the first means is provided which connects a line upstream of the first means and the first line of the sample preparation system (i.e. "step (p1)"). Preferably, the line upstream of the first means is the input line. In case that a clarification filter is present in the first means, said clarification filter is ejected before the flow-through path is provided. In an embodiment of the present invention, the connecting means comprised by the consumable handling device connects the input line and the first line for providing fluidic communication between the input line and the first line, thereby providing the flow-through path within the first means. This can for example be achieved by using the index wheel as the consumable handling device and rotating the index wheel to the second position of the index wheel where a through fluidic passage is located which can then connect to the input line and the first line.

Optionally, in case that the sample preparation system comprises the target substance detecting sensor, the sensor may be zeroed before the sample to be purified is provided to the purification filter or the purification chromatography column (i.e. "step (p2)"). For example, buffer such as PBS can be provided to the sample preparation system by a process fluid input valve arranged upstream of the first means. Preferably, the buffer is pumped through the third line of the sample preparation system and not through the second line of the sample preparation system.

Then, the sample to be purified can be provided to the input cup of the sample preparation system (i.e. "step (p3)"). Preferably, the pipetting robot can provide the sample to be purified to the input cup by transferring the sample to be purified from at least one input vessel to the input cup of the sample preparation system.

Afterwards, the sample to be purified can be pumped in the direction of the purification filter or the purification chromatography column (i.e. "step (p4)"). According to an embodiment, while the sample to be purified is pumped in the direction of the purification filter or the purification chromatography column, the first valve and the second valve are switched to bypass the second means for avoiding pumping excessive air into the purification filter or the purification chromatography column which might result in degraded performance and ultimately an air-lock blockage of the purification filter or the purification chromatography column. In an embodiment, when the sample to be purified is detected by the first liquid sensor, the first valve and the second valve are switched to direct the sample to be purified through the purification filter or the purification chromatography column. Preferably, when the sample to be purified is detected by the second liquid sensor, the first valve and the second valve are switched to direct the sample to be purified through the purification filter or the purification chromatography column. Thereby, even less air is pumped into the purification filter or the purification chromatography column. Optionally, after detection of the sample to be purified through either the first liquid sensor or the second liquid sensor, a predefined volume of the sample to be purified may be pumped prior to switching the first valve and the second valve and hence even less air or no air is pumped into the purification filter or the purification chromatography column.

In case that the input cup has emptied, the first liquid sensor can detect that the sample to be purified has run out (i.e. "step (p5)"). Specifically, the first liquid sensor can detect whether the sample to be purified previously provided to the input cup has already passed the location where the first liquid sensor is arranged.

Then, a rinsing step can be automatically carried out (i.e. "step (p6)"). In particular, a rinsing liquid such as PBS can be provided to the sample preparation system by a process fluid input valve arranged upstream of the first means. Thereby, sample to be purified still present upstream of the second means can be directed to the second means and purified by passing it through the purification filter or the purification chromatography column.

Subsequently, according to an embodiment, the target substance detecting sensor comprised by the sample preparation system may be zeroed after the rinsing step (i.e. "step (p7)"). For example, buffer such as elution buffer can be provided to the sample preparation system by a process fluid input valve arranged upstream of the first means. Preferably, the buffer is pumped through the third line of the sample preparation system and not through the second line of the sample preparation system.

Optionally, after step (p6) and/or step (p7), at least any liquid still contained in the third line of the sample preparation system can be drained (i.e. "step (p8)"). In an embodiment of step (p8), any liquid still present in the input line, the first line and/or the fourth line of the sample preparation system can be further drained. Thereby, air can be introduced into the third line which may be used for pushing sample to the output cup in a later step.

Then, according to an embodiment, an elution step is carried out (i.e. "step (p9)"). Preferably, while a buffer such as elution buffer is pumped in the direction of the purification filter or the purification chromatography column, the first valve and the second valve are switched to bypass the second means for avoiding pumping excessive air into the purification filter or the purification chromatography column which may result in degraded performance and ultimately an air-lock blockage of the purification filter or the purification chromatography column. In an embodiment, when the buffer such as elution buffer is detected at the first liquid sensor, the first valve and the second valve are switched to direct the buffer through the second line via the purification filter or the purification chromatography column. Preferably, when the buffer such as elution buffer is detected at the second liquid sensor, the first valve and the second valve are switched to direct the buffer through the second line via the purification filter or the purification chromatography column. Thereby, even less air is pumped into the purification filter or the purification chromatography column. Optionally, after detection of the buffer such as elution buffer through either the first liquid sensor or the second liquid sensor, a predefined volume of the buffer may be pumped prior to switching the first valve and the second valve and hence even less air or no air is pumped into the purification filter or the purification chromatography column. The buffer can be provided to the sample preparation system by a process fluid input valve arranged upstream of the first means. In an embodiment of step (p9), the elution buffer may be a mixture of a first elution buffer and a second elution buffer, wherein the first elution buffer can be provided to the sample preparation system by a first process fluid input valve arranged upstream of the first means and the second elution buffer can be provided to the sample preparation system by a second process fluid input valve arranged upstream of the first means. Thereby, gradient elution can be performed by controlled use of the first and second process fluid input valves.

Afterwards, the purified sample can be collected in the output cup (i.e. "step (p10)"). In an embodiment, the purified sample corresponds to a fraction that contains the target substance eluted from the purification filter or the purification chromatography column. Specifically, the fraction can be eluted by using the elution buffer in step (p9). According to an embodiment, the concentration of the target substance in the fraction is higher than 0.4 g/L, preferably higher than 0.5 g/L. Therefore, the fraction is suitable for direct analysis via various subsequent assays.

The purified sample can be collected by different methods in the above step (p10). According to an embodiment, the target substance detecting sensor can be used to detect when the target substance passes the sensor. In another embodiment, the sample may be purified by using a predetermined elution volume. In both embodiments, in case that a rinsing step (step (p6)) has been carried out before step (p10), the initial liquid emerging from the purification filter or the purification chromatography column is mostly buffer used in the rinsing step and contains very little target substance and is therefore preferably directed to the waste vessel. When the target substance detecting sensor detects the target substance or the appropriate amount of the predetermined elution volume has been eluted, the output valve is switched to direct the purified sample to the output cup. In a preferred embodiment, the target substance detecting sensor is a UV sensor. Then, the total eluted target substance can be calculated by using the output from the UV sensor, the volume of the elution buffer pumped through the system, the optical path length and the extinction coefficient of the target substance such as a protein.

In an embodiment, after sufficient buffer such as elution buffer has been pumped in the direction of the purification filter or the purification chromatography column, the first valve and the second valve can optionally be switched such that air possibly present in the third line of the sample preparation system provided in a previous step (i.e. of step (p8)) can be used to direct purified sample still present in the output line to the output cup (i.e. "step (p11)").

Preferably, the output sample present in the output cup can be transferred to at least one output vessel (i.e. "step (p12)"). This step can, for example, be carried out by the pipetting robot.

Optionally, the sample preparation system can then be cleaned by pumping cleaning liquids such as a buffer, sodium hydroxide and/or water through the sample preparation system from process fluid input valves arranged upstream of the first means to the output cup, then in reverse from the output cup to the input cup (i.e. "step (p13)"). The resulting liquid can then be directed to a waste vessel by the use of a waste pump.

According to an embodiment, the purification filter or the purification chromatography column is then removed from the sample preparation system, if it is at the end of its useful life (i.e. "step (p14)").

### Combination of the clarification process (i) and the purification process (ii)

In the method for preparing a sample using the sample preparation system comprising the clarification process (i) and the purification process (ii) (hereinafter referred to as "the combined method"), the clarification process (i) (i.e. step (i)) is carried out before the purification process (ii) (i.e. step (ii)).

The purification filter or the purification chromatography column used in the purification process (ii) of the combined method is preferably already present in the sample preparation system before the clarification process (i) begins. The purification filter or the purification chromatography column can for example be provided manually to the sample preparation system. In another embodiment, the purification filter or the purification chromatography column can be provided in an automated manner to the sample preparation system.

According to a preferred embodiment, the purification filter or the purification chromatography column is in an equilibrated state. The term "equilibrated state" used herein means that the purification filter or the purification chromatography column is in a state which allows binding the target substance. This state can for example be achieved by bringing the purification filter or the purification chromatography column to a certain pH such as 7 or higher while containing the binding compound. In case that the purification filter or the purification chromatography column is not in an equilibrated state, an equilibration buffer such as PBS can be pumped through the purification filter or the purification chromatography column. This step can be carried out at any point of the combined method before the below-described step (4)a or step (4)b.

Further, according to an embodiment, excess air can be removed from the upstream side of the purification filter or the purification chromatography column for avoiding an air-lock blockage of the purification filter or the purification chromatography column. In particular, a buffer such as PBS can be pumped to the output cup, and then the pump is reversed to pull the buffer through the purification filter or the purification chromatography column from its outlet to the inlet while monitoring and controlling the negative pressure at the inlet of the purification filter or the purification chromatography column by the second pressure sensor. In an embodiment, the pressure is controlled to be 0.4 bar absolute or higher. Then, after the equilibration step and/or the step of removing excess air from the purification filter or the purification chromatography column, the input cup, the output cup and the lines of the sample preparation system except the second line can be drained.

According to an embodiment, at the beginning of the clarification process (i) of the combined method, a clarification filter is provided to the first means, wherein the clarification filter is new, i.e. unused (hereinafter referred to as "step (1)"). In said step (1), preferably, the clarification filter is provided by the consumable handling device from the input stack.

In another embodiment, a clarification filter may already be connected to the first means at the beginning of the clarification process (i). In both embodiments, it might be necessary to exchange the clarification filter during the clarification process (i) for avoiding blockage of the clarification filter and product loss. According to an embodiment, the clarification filter is automatically ejected from the first means and automatically replaced by a new clarification filter. Preferably, based on the measured backpressure of the clarification filter measured by the first pressure sensor, or the pressure difference between the first pressure sensor and the second pressure sensor, and the pumping speed, a decision can be made to automatically eject the clarification filter from the first means and automatically replace it by a new clarification filter.

Then, the clarification filter is preferably clamped such that the upper face and the lower face of the clarification filter are supported against the internal pressure during filtration (i.e. "step (2)").

Further, an air pressure test can be performed on the sample preparation system and the clarification filter before the sample to be clarified and purified is pumped to the clarification filter (i.e. "step (3)"). In particular, in said step (3), the first valve and the second valve are set to close the flow path and the pump pumps air into the sample preparation system to reach a preset pressure. According to an embodiment, the preset pressure is from 0.1 bar to 4 bar, preferably from 0.5 bar to 1 bar. Then, the pressure is monitored for a few seconds. At the end of the air pressure test, the pressure is released by setting the first valve, the second valve and the output valve such that the pressure is released to the waste vessel via the third line.

After the optional air pressure test, the sample to be clarified and purified can be provided to the input cup of the sample preparation system (i.e. "step (4)a"). As an alternative, the sample to be clarified and purified can also be provided to the input cup of the sample preparation system before any of steps (1) to (3) (i.e. "step (4)b"). Preferably, the pipetting robot can provide the sample to be clarified and purified to the input cup by transferring the sample to be clarified and purified from at least one input vessel to the input cup of the sample preparation system.

Then, the sample to be clarified and purified can be pumped from the input cup to the clarification filter (i.e. "step (5)"). According to an embodiment of said step (5), the sample to be clarified and purified passes on its way from the input cup to the clarification filter at least the input sample valve, the first liquid sensor and the first pressure sensor.

Further, the sample is clarified by passing it through the clarification filter connected to the first means (hereinafter referred to as "step (6)").

Then, the clarified sample immediately flows from the clarification filter to the purification filter or the purification chromatography column (i.e. "step (7)"). Therefore, the clarified sample emerging from the clarification filter is not directly directed to the output cup, as described for an embodiment of the method for preparing a sample using the sample preparation system, wherein the method comprises a clarification process (i) but not the purification process (ii), but immediately to the purification filter or the purification chromatography column instead.

In case that the input cup has emptied, the first liquid sensor can detect that the sample to be clarified and purified has run out (i.e. "step (8)"). Specifically, the first liquid sensor can detect whether the sample to be clarified and purified previously provided to the input cup has already passed the location where the first liquid sensor is arranged.

After step (8), a rinsing step can be automatically carried out (i.e. "step (9)"). In particular, a rinsing liquid such as PBS can be provided to the sample preparation system by a process fluid input valve arranged upstream of the first means. Thereby, sample to be clarified and purified still present upstream of the first means can be directed to the first means and sample within the clarification filter can be rinsed through the clarification filter. Preferably, the rinsing liquid which passed through the clarification filter containing clarified sample is directed to the purification filter or the purification chromatography column. Thereby, clarified sample that needs to be purified still present upstream of the second means can be directed to the second means and purified by passing it through the purification filter or the purification chromatography column.

After step (8) and/or step (9), a step of pumping air is preferably carried out (i.e. "step (10)"). Specifically, air can be provided to the sample preparation system by a process fluid input valve arranged upstream of the first means. Air pushes the sample to be clarified and purified or rinse liquid still present on the upper flow field of the clarification filter and already partly clarified sample but not yet purified or rinse liquid still contained within the clarification filter through the clarification filter. The thereby yielded clarified sample can be directed to the purification filter or the purification chromatography column. Further, the clarified sample that needs to be purified can be pushed through the purification filter or the purification chromatography column by the step of pumping air. By applying step (10), product recovery can be increased. The air pressure used is for example in a range of from 0.5 bar to 2 bar. Thereby, the air pressure is below the bubble point of the clarification filter such that the creation of foam can be avoided. After a waiting time of for example from 0 s to 5 s, the air pressure is released by reversing the pump.

According to an embodiment of the combined method, the method further comprises between step (i) and step (ii), i.e. preferably at least after the above step (6), the below steps in the following order:
(a) ejecting the clarification filter,
(b) providing a flow-through path within the first means which connects a line upstream of the first means and the first line of the sample preparation system, and
(c) removing sample from the line upstream of the first means by directing said sample through the flow-through path, the first line and the third line of the sample preparation system.

In particular, in the above step (a), the clarification filter is ejected from the first means (i.e. "step (11)"). In case that the first means is the first filter holder, and the clarification filter is clamped by the first clamp and the second clamp, the clarification filter is unclamped before the clarification filter is ejected. The ejected clarification filter can be received by the consumable handling device and provided to the waste bin.

Further, according to an embodiment, the flow-through path of the above step (b) can be provided by using the connecting means comprised by the consumable handling device which connects the input line and the first line for providing fluidic communication between the input line and the first line such that the liquid path is closed (i.e. "step (12)"). This can for example be achieved by using the index wheel as the consumable handling device and rotating the index wheel to the second position of the index wheel where a through fluidic passage is located which can then connect to the input line and the first line.

Then, for removing the sample from the line upstream of the first means in accordance with the above step (c), a rinsing step can be carried out (i.e. "step (13)"). In particular, a rinsing liquid such as PBS can be provided to the sample preparation system by a process fluid input valve arranged upstream of the first means. Preferably, in this embodiment of step (c), the first valve and the second valve are switched to bypass the second means such that the rinsing liquid with unclarified sample can be directed through the flow-through path provided in step (b), the first line and the third line of the sample preparation system. Thereby, any unclarified sample still present upstream of the first means of the sample preparation system can be removed for avoiding contamination and blockage of the purification filter or the purification chromatography column. Further, in an embodiment, step (13) may optionally also be used for zeroing the target substance detecting sensor.

Afterwards, the first valve and the second valve can be switched to direct the rinsing liquid through the second line via the purification filter or the purification chromatography column (i.e. "step (14)"). Thereby, clarified sample that still needs to be purified present downstream of the first valve and upstream of the second means can be directed to the second means and purified by passing it through the purification filter or the purification chromatography column.

In an embodiment, after step (13) and/or step (14), at least any liquid still contained in the third line of the sample preparation system can be drained (i.e. "step (15)"). In an embodiment of step (15), any liquid still present in the input line and/or the first line of the sample preparation system can be further drained. Thereby, air can be introduced into the third line which may be used for pushing sample to the output cup in a later step.

Then, according to an embodiment, an elution step is carried out (i.e. "step (16)"). Preferably, while a buffer such as elution buffer is pumped in the direction of the purification filter or the purification chromatography column, the first valve and the second valve are switched to bypass the second means for avoiding pumping excessive air into the purification filter or the purification chromatography column which may result in degraded performance and ultimately an air-lock blockage of the purification filter or the purification chromatography column. In an embodiment, when the buffer such as elution buffer is detected at the first liquid sensor, the first valve and the second valve are switched to direct the buffer through the second line via the purification filter or the purification chromatography column. Preferably, when the buffer such as elution buffer is detected at the second liquid sensor, the first valve and the second valve are switched to direct the buffer through the second line via the purification filter or the purification chromatography column. Thereby, even less air is pumped into the purification filter or the purification chromatography column. Optionally, after detection of the buffer such as elution buffer through either the first liquid sensor or the second liquid sensor, a predefined volume of the buffer may be pumped prior to switching the first valve and the second valve and hence even less air or no air is pumped into the purification filter or the purification chromatography column. The buffer can be provided to the sample preparation system by a process fluid input valve arranged upstream of the first means. In an embodiment of step (16), the elution buffer may be a mixture of a first elution buffer and a second elution buffer, wherein the first elution buffer can be provided to the sample preparation system by a first process fluid input valve arranged upstream of the first means and the second elution buffer can be provided to the sample preparation system by a second process fluid input valve arranged upstream of the first means. Thereby, gradient elution can be performed by controlled use of the first and second process fluid input valves.

Afterwards, the clarified and purified sample can be collected in the output cup (i.e. "step (17)"). In an embodiment, the clarified and purified sample corresponds to a fraction that contains the target substance eluted from the purification filter or the purification chromatography column. Specifically, the fraction can be eluted by using the elution buffer in step (16). According to an embodiment, the concentration of the target substance in the fraction is higher than 0.4 g/L, preferably higher than 0.5 g/L. Therefore, the fraction is suitable for direct analysis via various subsequent assays.

The clarified and purified sample can be collected by different methods in the above step (17). According to an embodiment, the target substance detecting sensor can be used to detect when the target substance passes the sensor. In another embodiment, the collection of the clarified and purified sample may be based on a step using a predetermined elution volume. In both embodiments, in case that a rinsing step (step (13)) has been carried out before step (17), the initial liquid emerging from the purification filter or the purification chromatography column is mostly buffer used in the rinsing step and contains very little target substance and is therefore preferably directed to the waste vessel. When the target substance detecting sensor detects the target substance or the appropriate amount of the predetermined elution volume has been eluted, the output valve is switched to direct the clarified and purified sample to the output cup. In a preferred embodiment, the target substance detecting sensor is a UV sensor. Then, the total eluted target substance can be calculated by using the output from the UV sensor, the volume of the elution buffer pumped through the system, the optical path length and the extinction coefficient of the target substance such as a protein.

In an embodiment, after sufficient buffer such as elution buffer has been pumped in the direction of the purification filter or the purification chromatography column, the first valve and the second valve can optionally be switched such that air possibly present in the third line of the sample preparation system provided in a previous step (i.e. step (15)) can be used to direct the clarified and purified sample still present in the output line to the output cup (i.e. "step (18)").

Preferably, the output sample present in the output cup can be transferred to at least one output vessel (i.e. "step (19)"). This step can, for example, be carried out by the pipetting robot.

Optionally, the sample preparation system can then be cleaned by pumping cleaning liquids such as a buffer, sodium hydroxide and/or water through the sample preparation system from process fluid input valves arranged upstream of the first means to the output cup, then in reverse from the output cup to the input cup (i.e. "step (20)"). The resulting liquid can then be directed to a waste vessel by the use of a waste pump.

According to an embodiment, the purification filter or the purification chromatography column is then removed from the sample preparation system, if it is at the end of its useful life (i.e. "step (21)").

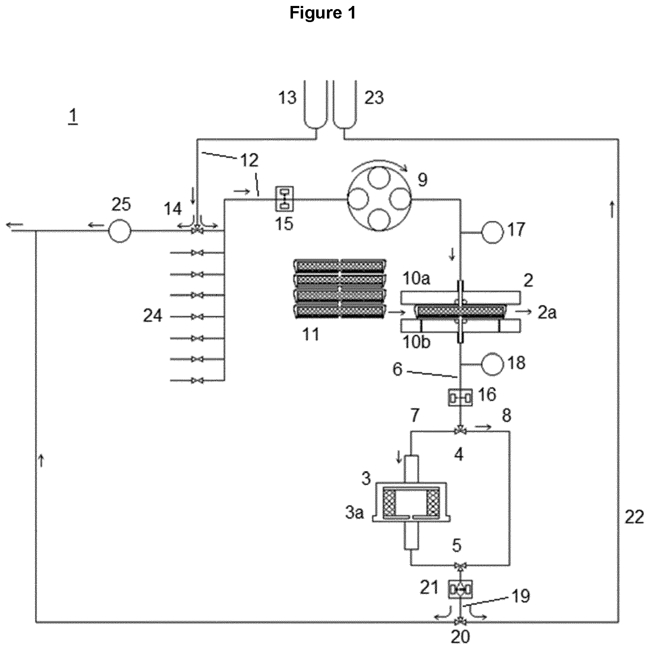

Figure 1 shows an embodiment of the sample preparation system of the present invention. In particular, the sample preparation system 1 comprises a serial arrangement of a first means 2 configured to being connected to a first consumable 2a and a second means 3 configured to being connected to a second consumable 3a, a first valve 4 arranged between the first means 2 and the second means 3, a second valve 5 arranged downstream of the second means 3, a first line 6 providing fluidic communication between the first means 2 and the first valve 4, a second line 7 providing fluidic communication between the first valve 4 and the second valve 5 via the second means 3, a third line 8 providing fluidic communication directly between the first valve 4 and the second valve 5 configured to bypass the second means 3, and a pump 9 configured to move fluid within the sample preparation system 1. A first consumable 2a is connected to the first means 2. A second consumable 3a is connected to the second means 3. The first means 2 is a first filter holder comprising a first clamp 10a configured to clamp the first consumable 2a on the upper face (i.e. upstream) and a second clamp 10b configured to clamp the first consumable 2a on the lower face (i.e. downstream). The sample preparation system 1 further comprises an input stack 11 holding first consumables 2a. The sample preparation system 1 further comprises an input line 12 providing fluidic communication between an input cup 13 arranged upstream of the first means 2 and the first means 2. An input sample valve 14 is located downstream of the input cup 13 and upstream of the first means 2 on the input line 12. Moreover, a first liquid sensor 15 is arranged downstream of the input sample valve 14 and upstream of the first means 2. A second liquid sensor 16 is arranged downstream of the first means 2 and upstream of the second means 3. A first pressure sensor 17 is arranged downstream of the first liquid sensor 15 and upstream of the first means 2. A second pressure sensor 18 is arranged downstream of the first means 2 and upstream of the second means 3. The sample preparation system 1 further comprises a fourth line 19 providing fluidic communication between the second valve 5 and an output valve 20 arranged downstream of the second valve 5. A target substance detecting sensor 21 is arranged downstream of the second valve 5 and upstream of the output valve 20. The sample preparation system 1 further comprises an output line 22 providing fluidic communication between the output valve 20 and an output cup 23. Further, the sample preparation system 1 comprises process fluid input valves 24 arranged upstream of the first means 2. The sample preparation system 1 is connected to an external waste vessel (not shown) to which waste can be directed by the use of a waste pump 25.

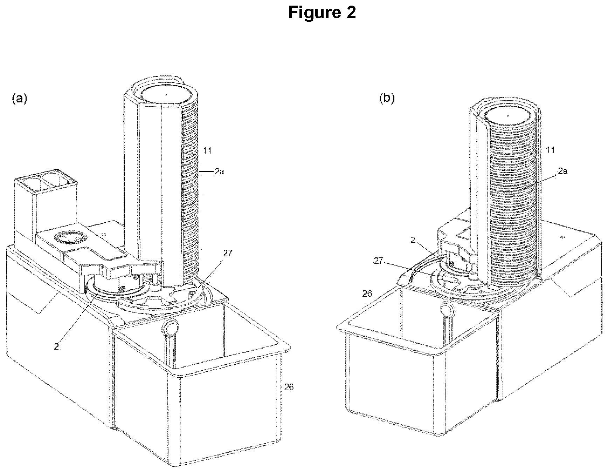

Figures 2 (a) and (b) show side views of an embodiment of a part of the sample preparation system of the present invention and an (external) waste bin 26. In particular, a consumable handling device 27 is shown which is an index wheel and connected to the first means 2 and to the input stack 11 holding first consumables 2a.

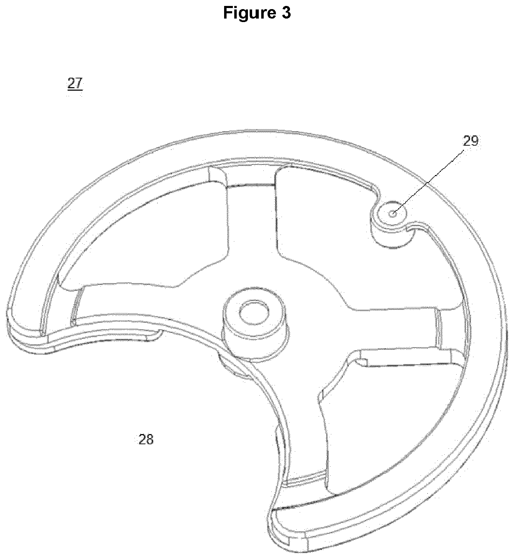

Figure 3 shows an embodiment of the consumable handling device 27 in the form of an index wheel. The index wheel has an opening 28 in its in-plane direction which conforms to the shape of a first consumable at a first position of the index wheel. The index wheel has a hole 29 (corresponding to the connecting means) in the in-plane direction of the index wheel that extends from the first surface of the index wheel to the second surface of the index wheel at a second position of the index wheel.

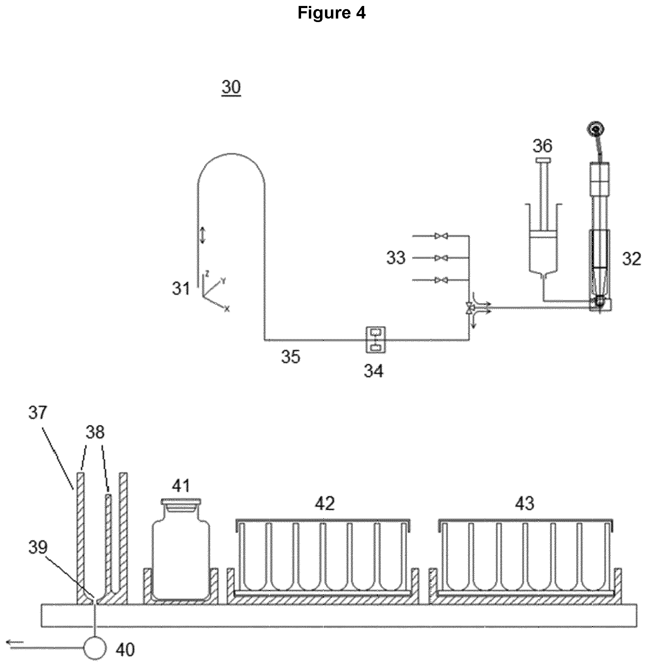

Figure 4 shows an embodiment of the pipetting robot (top) and an embodiment of the pipette wash station (bottom) that may be used in the present invention. In particular, the pipetting robot 30 comprises a pipette tip 31 connected to a pH electrode 32 and to fluid input valves 33. The pipetting robot 30 further comprises a pipette liquid sensor 34 arranged on a pipetting robot line 35 providing fluidic communication between the pipette tip 31, the pH electrode 32 and the fluid input valves 33. Moreover, the pipetting robot 30 comprises a syringe 36 connected to the pH electrode 32. In the embodiment shown in Figure 4, the pipette wash station 37 has walls 38. Further, the pipette wash station 37 has an outlet 39 at its bottom. The outlet 39 is connected to a waste vessel (not shown) to which liquid may be pumped by the use of a tip wash waste pump 40. Further shown in Figure 4 are a storage vessel 41 for storing for example process liquids, input vessels 42 and output vessels 43 which may be arranged next to the pipette wash station 37.

### List of Reference Numerals

- 1: sample preparation system
- 2: first means
- 2a: first consumable
- 3: second means
- 3a: second consumable
- 4: first valve
- 5: second valve
- 6: first line
- 7: second line
- 8: third line
- 9: pump
- 10a: first clamp
- 10b: second clamp
- 11: input stack
- 12: input line
- 13: input cup
- 14: input sample valve
- 15: first liquid sensor
- 16: second liquid sensor
- 17: first pressure sensor
- 18: second pressure sensor
- 19: fourth line
- 20: output valve
- 21: target substance detecting sensor
- 22: output line
- 23: output cup
- 24: process fluid input valves
- 25: waste pump
- 26: waste bin
- 27: consumable handling device
- 28: opening
- 29: hole
- 30: pipetting robot
- 31: pipette tip
- 32: pH electrode
- 33: fluid input valves
- 34: pipette liquid sensor
- 35: pipetting robot line
- 36: syringe
- 37: pipette wash station
- 38: walls
- 39: outlet
- 40: tip wash waste pump
- 41: storage vessel
- 42: input vessels
- 43: output vessels

## Claims

1. A sample preparation system comprising:
a serial arrangement of a first means configured to being connected to a first consumable and a second means configured to being connected to a second consumable;
a first valve arranged between the first means and the second means;
a second valve arranged downstream of the second means;
a first line providing fluidic communication between the first means and the first valve;
a second line providing fluidic communication between the first valve and the second valve via the second means;
a third line providing fluidic communication directly between the first valve and the second valve configured to bypass the second means; and
a pump configured to move fluid within the sample preparation system.

2. The sample preparation system according to claim 1,
wherein the first means is configured to being connected to the first consumable and to support the first consumable.

3. The sample preparation system according to claim 1 or 2,
wherein the second means is configured to being connected to the second consumable and to support the second consumable.

4. The sample preparation system according to any one of claims 1 to 3,
wherein the first consumable is a first filter.

5. The sample preparation system according to any one of claims 1 to 4,
wherein the second consumable is a second filter or a chromatography column.

6. The sample preparation system according to any one of claims 1 to 5,
wherein the first means is a first filter holder.

7. The sample preparation system according to any one of claims 1 to 6, further comprising:
a consumable handling device connected to the first means configured to provide a first consumable to the first means and to receive a first consumable from the first means.

8. The sample preparation system according to claim 7,
further comprising an input line providing fluidic communication between an input cup arranged upstream of the first means and the first means,
wherein the consumable handling device comprises a connecting means configured to connect the input line and the first line for providing fluidic communication between the input line and the first line.

9. The sample preparation system according to claim 8,
further comprising an input sample valve located downstream of the input cup and upstream of the first means on the input line.

10. The sample preparation system according to any one of claims 1 to 9,
further comprising a target substance detecting sensor arranged downstream of the second means configured to detect a target substance.

11. A method for preparing a sample using the sample preparation system according to any one of claims 1 to 10,
wherein the method comprises at least one of the following steps:
(i) clarifying the sample by passing it through a first consumable connected to the first means to provide a clarified sample, wherein the first consumable is a clarification filter and
(ii) purifying the sample by passing it through a second consumable connected to the second means to provide a purified sample, wherein the second consumable is a purification filter or a purification chromatography column.

12. The method for preparing a sample according to claim 11,
wherein the method comprises steps (i) and (ii), and step (i) is carried out before step (ii), wherein the clarified sample immediately flows from the clarification filter to the purification filter or the purification chromatography column.

13. The method for preparing a sample according to claim 12,
wherein the method further comprises between step (i) and step (ii), the below steps in the following order:
(a) ejecting the clarification filter,
(b) providing a flow-through path within the first means which connects a line upstream of the first means and the first line of the sample preparation system, and
(c) removing sample from the line upstream of the first means by directing said sample through the flow-through path, the first line and the third line of the sample preparation system.

14. The method for preparing a sample according to any one of claims 11 to 13,
wherein the sample is a cell culture comprising a protein,
the clarification filter is a cell culture clarification filter, and
the purification filter is a protein purification filter or a protein purification chromatography column.

15. The method for preparing a sample according to any one of claims 11 to 14, wherein, in step (i), the clarification filter is automatically ejected from the first means and automatically replaced by a new clarification filter.
